# EUROPEAN PATENT SPECIFICATION

(11) **EP 0 671 471 B1**
(45) Date of publication and mention of the grant of the patent: **07.04.1999**
(21) Application number: 94901001.1
(22) Date of filing: 25.11.1993
(51) Int. Cl.: C12P 21/00, C12P 21/08, C12N 15/85, C12N 15/06, C12N 5/20

(54) **PROCESS FOR PRODUCING FOREIGN PROTEIN**
HERSTELLUNGSPROZESS VON FREMDPROTEIN
PROCEDE DE PRODUCTION D'UNE PROTEINE ETRANGERE

(30) Priority: 28.11.1992 JP 341256/92
(43) Date of publication of application: 13.09.1995
(73) Proprietor: Juridical Foundation, The Chemo-Sero-Therapeutic Research Institute, Kumamoto-shi, Kumamoto-ken (JP)
(72) Inventor: NISHIYAMA, Kiyoto, Kikuchi-shi, Kumamoto 861-13 (JP); ISHIKAWA, Yuji, Kumamoto-shi, Kumamoto 862 (JP); KIMACHI, Kazuhiko, Kikuchi-gun, Kumamoto 861-11 (JP); MAEDA, Hiroaki, Kumamoto-shi, Kumamoto 862 (JP); TOKIYOSHI, Sachio, Kumamoto-shi, Kumamoto 862 (JP)
(74) Representative: Vossius, Tilman
(86) International application number: PCT/JP93/01723
(87) International publication number: WO 94/12658

(56) References cited:
- JP-A- 2 057 184
- JP-A- 2 503 514
- JP-A- 4 504 658
- JP-A-62 502 377
- US-A- 4 757 018
- US-A- 5 110 737
- J. MED. COLL. PLA (1991), 6(2), 135-40 , 1991, XP000574789 LI, JILIANG ET AL: "Cholesterol requirement for growth of rodent parental myeloma cells in serum-free medium"
- J GEN MICROBIOL 133 (12). 1987. 3581-3590, XP000574785 SAWADA S ET AL: "IMMUNOPROTECTIVE HUMAN MONOCLONAL ANTIBODIES AGAINST FIVE MAJOR SEROTYPES OF PSEUDOMONAS-AERUGINOSA."
- HYBRIDOMA 6 (6). 1987. 679-688, XP000574786 JAHN S ET AL: "CELL BIOLOGY OF HUMAN IGM-PRODUCING HYBRIDOMAS DERIVED FROM A FUSION OF HUMAN SPLEEN LYMPHOCYTES WITH MOUSE MYELOMA CELLS."
- DATABASE WPI Section Ch, Week 9138 Derwent Publications Ltd., London, GB; Class B04, AN 91-277581 XP002007427 & JP-A-03 183 477 (MORINAGA & CO LTD) , 9 August 1991
- Cytotechnology, Vol. 6, No. 2, (1991), K. IWAMOTO et al., "Improvement in the Proliterative Activity of Human-Human Hybridomas at Low Cell Density by Transtection with bFGF Gene", p. 93-103.
- International Journal of Cancer, Vol. 48, No. 4, (1991), R. LASKOV et al., "Suppression of the Translocated myc Gene and Expression of Intracisternal A-Particle Genes in Tumorigenic and Non-Tumorigenic Hybrids between Murine Myeloma and Normal Fibroblasts", p. 574-582.

## Description

The present invention relates to a method for the production of exogenous proteins which comprises efficient expression of an exogenous gene prepared by recombinant DNA technology and isolation of the expressed protein. Specifically, it relates to an excellent method for the production of exogenous protein wherein the desired exogenous protein, in particular a recombinant antibody, is produced by serum-free culture utilizing as a host cell for expression, a fused cell obtained by fusing a mouse myeloma with a lymphatic cell.

The progress in cell fusion techniques in recent years has simplified the production of monoclonal antibodies. However, in case of heterogenous antibodies other than a mouse-type antibody, when a heterogenous cell e.g. a human cell is fused with a myeloma cell line of rodents such as mouse, rat, etc., the phenomenon that chromosomes of said heterogenous antibody deleted rapidly in the hybrid cell (hybridoma) is observed. For this reason, it has been quite difficult to obtain a stable antibody-producing cell ; cf. J.M. Austyn et al., Eur. J. Immunol., Vol. 11 (1981), 805. Although cell fusion has been attempted by using a homogenous myeloma cell line or B cell line other than those derived from rodents, no satisfactory results have been obtained.

On the other hand, with the progress of recombinant DNA technology, cloning of genes has become easier. As regards antibody, expression techniques have been developed at the genetic level which made it possible to isolate and express an antibody gene in a suitable host cell without deletion of chromosomes. However, the amount of antibody expressed after transfection of an antibody gene was generally smaller than that of a hybrid cell prepared by a cell fusion technique because the expression system was not optimized, and hence, this expression method has not been practical. In order to solve this problem, a gene amplification system with e.g. a DHFR gene has been used (G. Urlaub et al., PNAS USA, Vol. 77 (1980), 4212-4220), but in case of a mouse-derived cell system, it has been shown that an exogenous gene is not incorporated into a host chromosome as DM (Double Minutes) and thus instable ; cf. G.R. Stark et al., Cell, Vol. 57, (1989), 901-908. There are several reports on a DHFR gene amplification system using a mouse cell but they do not refer to the stability of the antibody producing cells.

A technique for antibody production by recombinant DNA technology has not been satisfactory in view of an expression system practically usable at an industrial level. Thus, it is desired to develop an excellent method for producing a recombinant antibody both in view of the expression and purification of a recombinant antibody.

From the technical point of view the production of a recombinant antibody using a serum-free culture, is preferable. According to the present invention it has been found that the desired recombinant antibody is quite efficiently prepared by using, as a host cell, a fused cell which is prepared from a mouse myeloma and a lymphatic cell and which can be cultured in serum-free medium (although such a cell may also be defined as a hybrid cell, in the present invention, the characteristic hybrid cell used as a host cell for expression of an exogenous protein is referred to as a fused cell) and expressing an antibody gene in said fused cell.

Several different methods have been reported which use transfection of recombinant cells into mouse myeloma cells. ; cf. Beidler et al., J. Immunol., Vol. 141 (1998), 4053-4060, and Neumaier et al., Cancer Res., Vol. 50 (1990), 2128-2134. In the hitherto known methods, however, a parent cell line (myeloma) itself is used as a host cell instead of being used for preparing a hybridoma, an exogenous gene is incorporated into said host cell and the antibody is expressed. However, when a mouse myeloma cell is used as a host cell for expression of an exogenous gene, it is difficult to prepare cells suitable for culture in serum-free medium and it is considered to be quite difficult to prepare a host cell which is both capable of growing in a serum-free medium like in a medium supplemented with serum and shows a stable gene expression efficiency.

A representative mouse myeloma cell is the P3X63Ag8.653 cell line. This cell line is an excellent host cell since an exogenous gene can easily be transfected into the cell line, integrated into the genome and it shows a high expression efficiency. However, as mentioned above, the P3X63Ag8.653 cell line is not easily adapted to a serum-free medium, its growth is variable depending on the transformant, and hence it can not grow in serum-free medium but can only grow in a culture medium supplemented with serum. According to the present inventors' studies, this cell line itself shows a cholesterol-dependent autotrophy and the culture medium needs to be supplemented with LDL, YLP (yoke lipoprotein), liposome, etc. for a long subculture. However, even in these culture media containing a high amount of cholesterol, growth is not completely equivalent to that in a medium supplemented with serum. Other unknown factors derived from serum are still required. So far, growth factors in the serum which are required by said cell are still unknown.

Thus, the present inventors have fused such mouse myeloma cells with lymphatic cells to prepare fused cells which are adapted so as to be able to be cultured in serum-free medium, and used the thus prepared fused cells as host cells for expression of an exogenous gene. As a result, it was found that transducing cells which express a desired exogenous gene efficiently and stably and which are capable of being cultured even in serum-free medium can be prepared. The lymphatic cells used in the present invention for preparing such fused cells need to be cells having a property of adaptability to serum-free culture after fusion. For example, among hybrid cells (hybridomas) generally prepared for the production of a monoclonal antibody from a myeloma cell and a lymphatic cell, those cells that were adapted or adaptable to serum-free culture can effectively be used. Among such hybrid cells, a suitable cell for fusion with a mouse myeloma in the present invention includes the hybrid cell Sp2/0 (ATCC No. CRL1581).

The most preferable mouse myeloma to be used for preparation of a fused cell in the present invention includes the mouse myeloma P3X63Ag8.653 cell line (ATCC No. CRL1580). The present inventors have studied in various ways a cell line for the use in a preparation of a fused cell being capable of efficiently expressing a recombinant antibody gene, and as a result, confirmed that the mouse myeloma P3X63Ag8.653 cell line is the most preferred among the various mouse myelomas in view of introduction of an exogenous gene.

In order to achieve the production of a large amount of an exogenous protein, e.g. a recombinant antibody, on an industrial level, the following conditions should be met:
(1) It should grow in a serum-free medium of low cost in which a desired product can easily be purifired;
(2) It should produce a large amount of a desired product; and
(3) It should easily be scaled up in a suspension culture system.

The method for expression of an exogenous gene of the present invention using, as a host cell for expression of the exogenous gene, a fused cell capable of being cultured in serum-free medium prepared by fusing a mouse myeloma with a lymphatic cell is characterized by fused cells, which are obtained by mixing a mouse myeloma (e.g. P3X63Ag8.653 cell line) with a lymphatic cell and conducting a cell fusion by adding a fusing agent such as polyethylene glycol. The fused cells are subjected to selection in a serum-free medium and to a series of cloning steps to prepare fused cells which can grow in serum-free medium and which can stably express an exogenous antibody gene. Said fused cells are used as host cells. These cells are an excellent tool for the production of an exogenous protein. The desired exogenous gene can be expressed in said fused cell and thereby the desired exogenous protein can be collected from serum-free medium. The method of the present invention satisfies all the above-mentioned conditions.

The kind of exogenous protein to be prepared by the method of the present invention is not specifically limited and includes various useful proteins, for example, antigenic proteins, physiologically active proteins and enzymes, but in particular, it is extremely useful for the production of an antibody by recombinant DNA technology. These cells are extremely suitable for expression of various genetically recombined antibodies such as a chimeric antibody, a V region-reshaped antibody [CDR reshaping antibody] etc., and can efficiently produce a desired recombinant antibody.

### Brief Explanation of Drawings

Figure 1 shows the structure of a vector expressing the IgG-L chain by way of an example of an exogenous gene used for a gene transfection test.

Figure 2 shows the structure of a vector expressing the IgG-H chain used for transformation in Example 4.

Figure 3 shows the structure of a vector expressing the IgG-L chain used for transformation in Example 4.

Figure 4 shows the growth in serum-free medium of a transformant established by using a fused cell and the expression level of the recombinant antibody.

The present invention is explained in more detail hereinbelow by an example for the production of a recombinant antibody.

Generally, the basic composition of a culture medium used for suspension culture of mammalian cells, RPMI-1640 medium supplemented with 10% fetal calf serum, etc. is used. In a serum-free medium having a decreased amount of total protein as shown hereinbelow, the fused cell prepared in accordance with the present invention has a cell growth and a capacity to produce a recombinant antibody equivalent to those in a medium supplemented with serum.

A mixture of Ham F12 medium: Dulbecco modified MEM medium: RPMI1640 medium in a ratio of 1:1:2 or 2:1:1 is used as the basic medium and additives as shown in the following Table 1 are added thereto.

**Table 1**

| Additive | Final Concentration |
|---|---|
| Transferrin | 1.0 mg/ml |
| Ethanolamine | 0.020 mM |
| 2-Mercaptoethanol | 0.025 mM |
| Selenious acid | 0.013 mg/l |
| Antibiotics | q.s. |
| Mixture of vitamins | |
| Biotin | 2.0 mg/l |
| Calcium D-pantothenate | 2.0 |
| Choline chloride | 2.0 |
| Folic acid | 2.0 |
| Inositol | 4.0 |
| Nicotinamide | 2.0 |
| Pyridoxine hydrochloride | 2.0 |
| Riboflavin | 0.2 |
| Thiamin hydrochloride | 2.0 |

When a DHFR gene amplification system is used, about 10⁻⁷ M methotrexate (MTX) may be added to the medium, and when a G418 resistance gene is incorporated, about 0.5 mg/ml G418 may be added to the medium.

As lymphatic cells used in the present invention, normal lymphatic cells obtained from peripheral lymphocytes, lymph nodes or a spleen may be used, but also a lymphatic hybrid cell, typically Sp2/0, is suitable. Cell fusion is conducted by mixing such lymphatic cells with mouse myeloma cells (preferably P3X63Ag8.653) in the presence of an agent for cell fusion (e.g. polyethylene glycol, etc.) at room temperature. The cell fusion is conducted in the usual manner. For example, 1 to 10 x 10⁷ cells of P3X63Ag8.653 are mixed with 1 to 5 x 10⁸ cells of a normal lymphatic cell or with 1 to 10 x 10⁷ cells of Sp2/0 and the mixture is centrifuged at 1500 rpm for 5 minutes to precipitate the cells. After adding serum-free RPMI1640 medium (40 ml), centrifugation is conducted at 1500 rpm for 10 minutes to wash cells. After dissolving the cell pellet, 45% polyethylene glycol (having a degree of polymerization of 1500 to 4000) is added dropwise at a rate of 1 ml per minute and the mixture is stirred gradually for 1 minute. After further adding dropwise 1 ml of RPMI1640 medium over 1 minute and again adding dropwise additional 1 ml of the medium over 1 minute, PEG (polyethylene glycol) is diluted with 8 ml of RPMI1640 medium. The cell suspension is centrifuged at 1200 rpm for 10 minutes to collect the cells. After cell fusion, culture is carried out in serum-free medium containing 1% fetal calf serum for about 1 week and then in the above-mentioned completely serum-free medium so that the P3X63Ag8.653 cells which did not fuse die whereas fused cells continue to grow. In this way, a fused cell capable of growing in a serum-free medium can easily be obtained and antibody in the culture supernatant can be screened by a suitable assay such as an antibody labelled with a radioisotope or EIA using an enzyme-labelled antibody etc.

The present invention aims at expressing an exogenous gene, especially a recombinant antibody gene, and hence, a fused cell which does not secrete an antibody derived from the host can be sorted out among said fused cells capable of growing in serum-free medium by the above-mentioned screening method. Whether the fused cells are suited as a host for a recombinant protein, however, can be confirmed by conducting the following gene transfection test.

### Gene transfection test

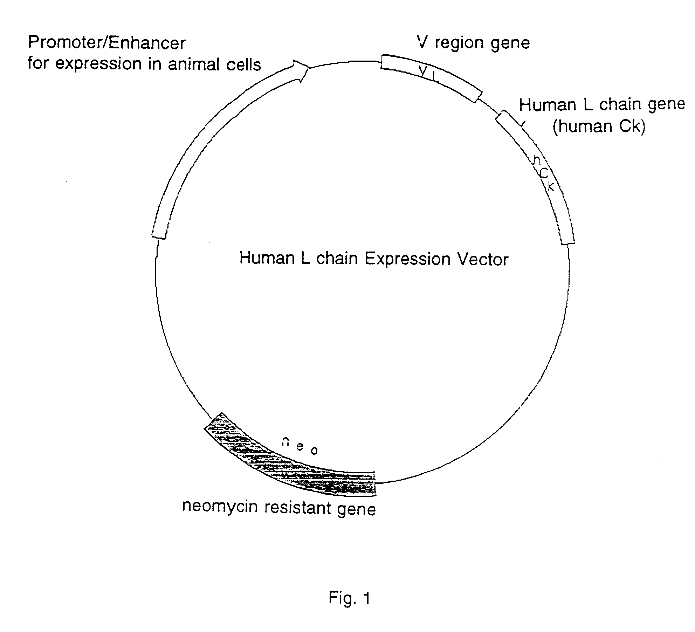

After conducting cell fusion in the usual manner and inoculating the suspension of the fused cells in a serum-free medium on a 24-well culture plate, culture is continued for about 2 months while exchanging culture medium with fresh serum-free medium for every 3 days. During this culture period, a portion of culture supernatant is taken out and the production of antibody is examined by EIA etc. at 3rd, 14th and 21st day after cell fusion. After about 10 days, pools of fused cells capable of growing in serum-free medium are obtained and wells showing good growth are selected and subjected to cloning by a limiting dilution method in RPMI1640 medium containing 10% fetal calf serum. Wells of a single clone are selected and expanded from a 96-well plate to a 24-well plate. When the clones show growth, first the culture medium is changed to serum-free medium containing 5% fetal calf serum and thereafter the concentration of fetal calf serum is gradually decreased and finally switched to serum-free medium. The cell density is counted and the cells are inoculated on another 24-well plate at a density of 2 x 10⁵ cells/ml and subjected to a gene transfection test. As to the remaining cells, culture is continued in serum-free medium until test results are obtained. The gene transfection test is conducted as follows: The 24-well plate is left to stand until the inoculated cells on the plate precipitate to the bottom of the well, and 0.3 ml of supernatant is removed to set the volume to 0.3 ml/well. A suitable expression plasmid (e.g. a plasmid vector for expression of an antibody L chain, etc. Fig. 1) is dissolved in sterile distilled water and mixed with a suitable agent for gene transfection (DEAE dextran, calcium phosphate, lipofectin, etc.) to give a total volume of 0.3 ml. After being left to stand at room temperature for 15 minutes, 0.01 ml of the said mixture is added dropwise to the cells on the 24-well plate and the cells are cultured at 37°C for 5 to 24 hours. After completion of culture, 0.3 ml of a serum-free medium supplemented with 20% fetal calf serum is added and the culture is further continued for 2 to 3 days and then the culture supernatant is studied for expression of the gene by using a suitable assay method.

A pool of fused cells which shows high expression efficiency of a desired product is selected and subjected to further cloning by a limiting dilution method using a 96-well microplate. The same gene transfection test is repeated to obtain a fused cell clone suitable as a host for recombinant DNA vectors.

In addition to the method of introducing an exogenous gene into the obtained fused cell line as mentioned above, it is also possible to first introduce an exogenous gene into a mouse myeloma. Then the obtained transfectant is fused with a normal lymphatic cell or another hybrid cell to prepare the genetic recombinant cell of the present invention which is capable of growing in serum-free medium. Thus, introduction of an exogenous gene can be conducted either prior to or after the step of obtaining a fused cell. If the introduction of an exogenous gene is carried out prior to cell fusion, selection in a serum-free medium is conducted first to destroy cells derived from a mouse myeloma (e.g. F3X63Ag8.653). Then culture in a selection medium (containing MTX, G418) supplemented with 1% fetal calf serum is conducted to give antibody-producing cells. The thus obtained cells can gradually be adapted to a serum-free medium. If a large number of transformants are desired, the method of introducing an exogenous gene after cell fusion is more convenient in order to save labor in the cell fusion step.

When the Sp2/0 cell line is employed as a partner for cell fusion, the G418 resistance gene expression unit etc. may in advance be introduced into the mouse myeloma P3X63Ag8.653 as a selection marker for the fusion so that fused cells can easily be selected by culture in a suitable selection medium after fusion. With the use of these agents for gene introduction, it was confirmed that the mouse myeloma P3X63Ag8.653 effectively integrates an exogenous gene whereas Sp2/0 cells themselves hardly integrate an exogenous gene at all.

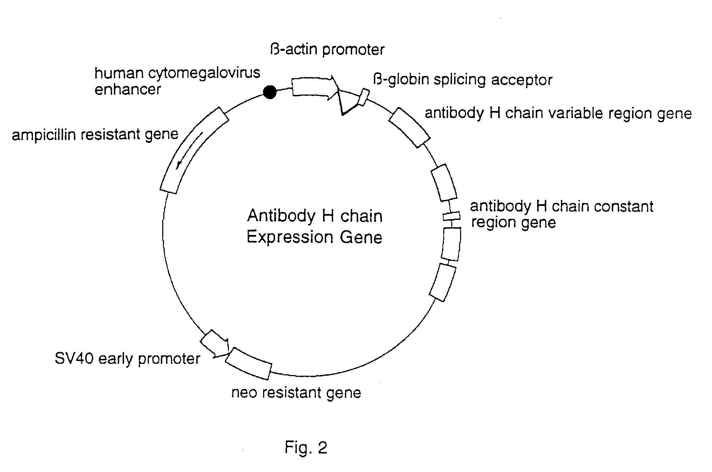

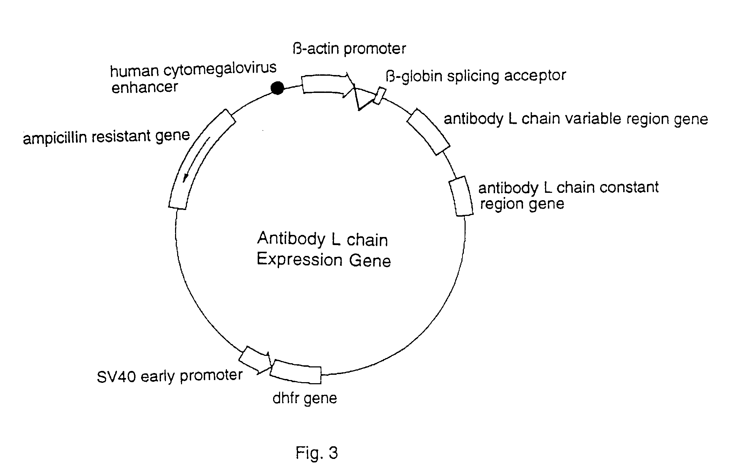

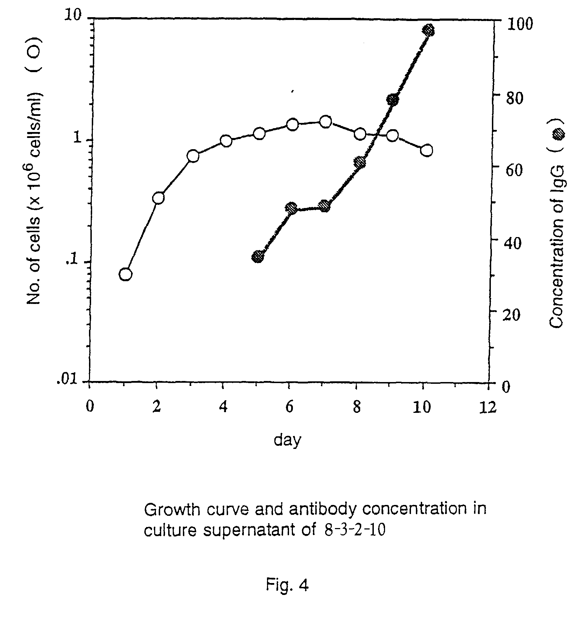

For use as a host cell for gene transfection, the ability to produce a large amount of a desired gene product is another important factor to be considered in addition to a good transformation efficiency and capability of being cultured in serum-free medium. Accordingly, it is important to assay the expression capacity of the host cell in advance. Those cells which have the highest expression capacity should be selected as host cells. The expression capacity of a cell can be assayed by introducing a suitable gene into the cell by preparing transformants and then measuring the amount of an expressed product of said gene in the culture supernatant, on the cell surface or within the cell of the transformant. For example, Table 2 shows results obtained by an enzyme immunoassay which measures the amount of a recombinant protein expressed in the culture supernatant of transformants which are prepared by transforming 26 clones of fused cells and their parent cells (P3X63Ag8.653 and Sp2/0) with plasmids as shown in Fig. 2 and Fig. 3 followed by selection with G418. Most of the 26 clones of fused cells showed higher expression capacity than their parent cells and especially clone No. 34 cell showed the highest production amount. Thus, clone No. 34 cell was considered to be most suitable clone as a host cell for expression of an exogenous gene. Using this clone as a host cell, the expression gene as shown in Fig. 4 was expressed to produce as much as 20 pg antibody per cell per day per ml.

Hitherto, for expression of a large amount of antibody molecules in a mouse myeloma cell system, other cell culture techniques such as gene amplification etc. have been required. For example, Gillies et al. (BioTechnology, 7, p799 (1989)) introduced the gene of anti-tetanus toxin antibody into SP2/0 cells and conducted a gene amplification with MTX to increase the production amount by several times. The amount of antibody expressed by this cell was about 20 pg/cell/day/ml. Bebbington et al. (BioTechnology, 10, p169 (1992)) also carried out expression of an antibody gene in a myeloma cell and obtained transformants having 9.5 to 15 pg/cell/day/ml of production capacity, which was also attained by utilizing the glutamine synthetase gene amplification.

Gene amplification is disadvantageous because first it takes time for amplification, usually several months for obtaining a cell line with high expression. A second disadvantage is the lack of stability of gene expression, i.e. deletion of a gene occurred when cells were cultured without a selection reagent such as MTX or MSX, resulting in a decrease of the production amount to the level prior to the amplification. Thus, it has been necessary either to add these selection reagents during the culture period or to clone a stable cell line.

On the contrary, the transformed cell derived from the fused cell established in accordance with the present invention had an expression capacity equivalent to that of the above-mentioned established cell lines without need of gene amplification.- Hitherto, the present inventors have carried out expression of an antibody gene in P3-X63Ag8.653 or Sp2/0 cells and have found that those cells could express an antibody as low as at several pg/cell/day/ml or less. The present inventors surprisingly found that the fused cell prepared in accordance with the present invention has a high production capacity without gene amplification, and that this fused cell for the first time allows such a high production capacity. Accordingly, by using this fused cell for transformation, a cell having a high production capacity can easily be obtained without need of gene amplification.

It is also possible to further increase the expression level of the product of these cells by gene amplification using reagents such as MTX or MSX. For example, as shown in Example 6, gene amplification was conducted by increasing the MTX concentration during culture of the transformants, and as a result, an increase in expression of a recombinant protein by almost 10 times was obtained.

The present invention is explained in more detail hereinbelow by means of Examples but it should not be construed to be limited thereto.

### Example 1

The spleen was from a mouse with was not immunized. Spleen cells were sterily suspended in 10 ml of serum-free RPMI1640 medium. They were transferred to a centrifuge tube to precipitate the connective tissue etc. and the cell suspension was collected. To the spleen cell precipitate 0.5 ml of a growth medium containing fetal calf serum was added and further 10 ml of 10 times-dilution medium was added quickly and erythrocytes were removed by hypotension treatment at room temperature for 15 to 20 seconds. After hypotension treatment, 10 ml of a two-fold concentrated medium was quickly added, the cells were well suspended with a pipet, additional 10 or 20 ml of growth medium was added and the mixture was centrifuged (1500 rpm, 5 minutes) to give a precipitate of a spleen cell. Thereto was added 20 to 30 mL of growth medium and the mixture was well mixed with a pipet and then centrifuged (1500 rpm, 5 minutes, at room temperature) to obtain normal lymphatic cells. The mouse-derived spleen cells (1 to 5 x 10⁸ cells) and P3X63Ag8.653 cell line (1 to 10 x 10⁷ cells) were mixed at a ratio of 5 to 10:1. Thereto was added 40 mL of serum-free RPMI1640 medium and the mixture was centrifuged at 1500 rpm for 10 minutes to wash the cells. The cell precipitate was well suspended with a pipet and then 45% polyethylene glycol (degree of polymerization of 1500 to 4000) was added dropwise at a rate of 1 ml per minute and the mixture was stirred slowly for 1 minute. Then, 1 ml of RPMI1640 medium was added dropwise over 1 minute and additional 1 ml of the medium was added dropwise over 1 minute, and then PEG (polyethylene glycol) was diluted with 8 ml of RPMI1640 medium and a centrifugation was conducted at 1200 rpm for 10 minutes to collect the cells. After cell fusion, the culture was carried out in HAT medium (containing hypoxanthine, aminopterin and thymidine) supplemented with 10% fetal calf serum for several weeks and then in the above-mentioned completely serum-free medium. As a result, P3.653 cells which did not fuse died gradually whereas the fused cells continued to grow without destruction. As a result, the fused cells capable of growing in serum-free medium could be obtained.

### Example 2

A transfectant previously prepared by introducing an antibody expression vector containing a DHFR gene expression unit and a G418 resistance gene expression unit in the P3X63Ag8.653 cell line was cell-fused with Sp2/0 cell line (ATCC No. 1581) and selection in serum-free medium was conducted. The selected fused cells were further cultured in the above-mentioned serum-free medium containing MTX (methotrexate), and after 2 weeks, the presence of antibody in the culture supernatant was determined by EIA using a peroxidase-labelled anti-IgG antibody. The transfectants which did not fuse could not grow in the serum-free medium and died. On the other hand, the Sp2/0 cells which did not fuse could also not grow in the serum-free medium containing MTX and G418 and died too. The fused cells could survive in the serum-free medium containing MTX, and as a result, clones expressing large amounts of the desired antibody could be obtained quite efficiently. Moreover, the cloning in Example 1 was time-consuming since the fused cells had to be cultured in a selection medium such as HAT medium etc. whereas in Example 2, desired clones could be obtained in a short period of time.

### Example 3

The mouse hybridoma Sp2/0 cell line is easily adapted to serum-free medium, and hence, allows serum-free culture on an industrial scale. Furthermore, since the Sp2/0 cell line does not secrete an antibody, a fusion of Sp2/0 with P3X63Ag8.653 can provide a host for the introduction of an exogenous gene having the characteristics of both cell lines, i.e. the growth capacity of Sp2/0 and the high gene introduction rate and the high expression efficiency of P3X63Ag8.653.

After cell fusion of both cell lines in accordance with the method of the present invention, the culture was carried out in serum-free medium on a 24-well plate for about 2 months with changes of the culture medium. Pools showing good growth were selected and cloning was conducted in RPMI1640 medium containing 10% fetal calf serum. Wells with a single clone of fused cells were selected and expanded from a 96-well plate to a 24-well plate. When growth in serum-free medium containing 5% fetal calf serum was observed, the culture medium was exchanged to the completely serum-free medium. The cell number was counted for the obtained fused cell clone and the gene introduction test was conducted using lipofectin. As a result, those fused cells which could grow in serum-free medium showed an expression efficiency equivalent to that of the P3X63Ag8.653 cell line (Table 2). As a control, P3X63Ag8.653 itself could not grow in the serum-free medium and Sp2/0 could grow but showed an extremely low capacity of antibody production.

**Table 2**

| Clone No. | Secondary screening Anti-hCkEIA: Growth | No. of colony in G418-containing medium |
|---|---|---|
| 6 | .796 +++ | 20 |
| 7 | .174 | 3 |
| 8 | .426 | 14 |
| 10 | .271 | 16 |
| 13 | .188 | 3 |
| 14 | .616 | 3 |
| 16 | .143 | 1 |
| 17 | .053 | 0 |
| 18 | .363 | 0 |
| 20 | .379 +++ | 7 |
| 23 | .065 | 0 |
| 25 | .131 | 2 |
| 27 | .690 | 2 |
| 30 | .111 | 0 |
| 31 | .448 | 18 |
| 33 | .894 | 13 |
| 34 | 1.529 | 19 |
| 37 | .157 | 0 |
| 38 | .128 +++ | 9 |
| 40 | .336 | 5 |
| 41 | .059 | 0 |
| 44 | .413 | 0 |
| 45 | .720 +++ | 6 |
| 47 | .031 | 0 |
| 50 | .835 | 10 |
| 51 | .274 | 9 |
| P3X63Ag8.653 | .069 | 0 |
| Sp2/0 | .085 | 0 |

Using the fused cells obtained hereinabove, a recombinant antibody gene with a DHFR gene incorporated therein was prepared and the stability of antibody production was studied. The stability of antibody production varies with each clone, but comparatively stable clones among the above-mentioned clones did not show decrease in capacity of antibody production even after a large scale culture up to 600 l was conducted in the presence of MTX.

### Example 4

The fused cells obtained in Example 3 were tested for the expression capacity of a recombinant protein. Fig. 2 and Fig. 3 show the structure of the expression vectors coding for a recombinant antibody H chain and L chain. Among the fused cells obtained in Example 3, clone No. 34 was transfected with these two kinds of expression vectors. 10 µg of each antibody H and L chain expression plasmid were mixed with 50 µl of Lipofectin (BRL) and 10⁶ cells were transfected with the mixture. On day 3, transfectants were selected in RPMI1640 medium containing 1 mg/ml G418, 0.25 x 10⁻⁷ M MTX and 5% fetal calf serum. After selection, transfected cells were cloned by a limiting dilution method to give an antibody-producing cell line (named "8-3-2-10").

This cell line was cultured in serum-free medium and was found to grow well. During the culture on a small scale, the maximum cell density reached was 1.5 x 10⁶/ml (survival rate 92%) and the time required for 2-fold multiplication was about 16 hours.

### Example 5

A large scale culture of 600 liters in serum-free medium was attempted for 8-3-2-10 cells obtained in Example 4. Fig. 4 shows the growth curve of the cells and increase of the antibody levels expressed in the culture supernatant. This cell line could grow well even in serum-free medium and the maximum cell density reached was 1.45 x 10⁶ cells/ml. The amount of antibody produced by one cell per day was 20 pg/ml.

### Example 6

By employing the fused cells obtained in Example 3, the gene amplification capacity of the transfected cells was examined. The expression vectors coding for the recombinant antibody H chain and L chain were introduced into the clone No. 34, 10 µg of each of the expression plasmids, pSV2-neo containing the gene coding for the antibody H chain and pSV2-dhfr containing the gene coding for the antibody L chain, were mixed with 50 µl of Lipofectin (BRL) and 10⁶ cells of the fused cell were transfected with the mixture. On day 3, transfectants were selected in RPMI1640 medium containing 1 mg/ml G418, 0.25 x 10⁻⁷ M MTX and 5% fetal calf serum. After selection, the transfected cells were cloned by a limiting dilution method to give the antibody-producing cells SP119-2, 3, 7, 10, 15 and 16.

Then, these six clones were cultured in RPMI1640 medium containing 1 mg/ml G418, 1 x 10⁻⁷ M MTX and 5% fetal calf serum. After one month, the antibody concentration expressed in the culture supernatant of the resistant cells was measured by an enzyme immunoassay and compared with the antibody concentration obtained prior to gene amplification. The results are shown in the following Table 3.

**Table 3**

| Name of cell | Rate of increase in antibody production^{a)} |
|---|---|
| SP119-2 | 1.42 |
| 3 | 2.81 |
| 7 | 7.42 |
| 10 | 2.50 |
| 15 | 10.47 |
| 16 | 1.05 |

| | |
|---|---|
| a) Ratio of the expression level of 1 x 10⁻⁷ M MTX resistant cells to that of 0.25 x 10⁻⁷ M MTX resistant cells | |

As can be concluded from the above results, by increasing the MTX level in the medium by 4-fold and conducting gene amplification, an increase of the antibody expression level by 10-fold in the highest case was observed.

These experiments confirmed that clone No. 34 can be used for gene amplification.

Accordingly, a higher expression level can be expected by gene amplification of 8-2-3-10 cell as shown in Examples 4 and 5.

### Industrial Applicability of the Invention

For expression of an exogenous gene, especially a recombinant antibody gene, the method of the present invention, by serum-free culture, allows for the stable production of large amounts of a desired recombinant antibody etc. in serum-free medium and makes it quite easy to purify a desired exogenous protein, and hence, it provides an excellent novel method for production of an exogenous protein.

## Claims

1. A method for the production of an exogenous protein which comprises employing, as a host cell for expression, a fused cell which is prepared by fusing a mouse myeloma and a lymphatic cell and which can be cultured in serum-free medium, wherein said exogenous protein producing gene is incorporated into a mouse myeloma prior to cell fusion or after cell fusion culturing said fused cell in serum-free medium to express the gene and collecting said exogenous protein from said medium.

2. The method according to claim 1 wherein the mouse myeloma is the P3X63Ag8.653 cell line.

3. The method according to claim 1 wherein the lymphatic cell is a hybrid cell (hybridoma) which is prepared from a myeloma and a lymphatic cell and which can be cultured in serum-free medium.

4. The method according to claim 3 wherein the hybrid cell which can be cultured in serum-free medium is the hybrid cell line Sp2/0.

5. The method according to any one of claims 1 to 4 wherein the exogenous protein is a recombinant antibody.

6. The method according to claim 5 wherein an amino acid sequence of the recombinant antibody comprises, at least as a portion thereof, an amino acid sequence which is derived from an antibody of an animal other than a mouse.

## Patentansprüche

1. Ein Verfahren zur Herstellung eines exogenen Proteins, umfassend die Verwendung einer fusionierten Zelle als eine Wirtszelle für die Expression, wobei die fusionierte Zelle hergestellt wird durch Fusion eines Maus-Myeloms und einer lymphatischen Zelle und in einem Serum-freien Medium kultiviert werden kann, wobei das das exogene Protein produzierende Gen eingegliedert ist in ein Maus-Myelom vor der Zellfusion oder nach der Zellfusion, Kultivieren der fusionierten Zelle im Serum-freien Medium, um das Gen zu exprimieren und Sammeln des exogenen Proteins aus dem Medium.

2. Das Verfahren gemäß Anspruch 1, wobei das Maus-Myelom die P3X63Ag8.653-Zellinie ist.

3. Das Verfahren gemäß Anspruch 1, wobei die lymphatische Zelle eine Hybridzelle (Hybridoma) ist, die hergestellt ist aus einem Myelom und einer lymphatischen Zelle und die in Serum-freiem Medium kultiviert werden kann.

4. Verfahren gemäß Anspruch 3, wobei die Hybridzelle, die in Serum-freiem Medium kultiviert werden kann, die Hybridzellinie Sp2/0 ist.

5. Das Verfahren gemäß einem der Ansprüche 1 bis 4, wobei das exogene Protein ein rekombinanter Antikörper ist.

6. Das Verfahren gemäß Anspruch 5, wobei eine Aminosäuresequenz des rekombinanten Antikörpers mindestens als einen Teil hiervon eine Aminosäuresequenz umfaßt, die abgeleitet ist von einem Antikörper eines anderen Tieres als einer Maus.

## Revendications

1. Procédé de production d'une protéine exogène dans lequel on utilise, comme cellule hôte pour l'expression, une cellule fusionnée préparée par fusion d'un myélome de souris et d'une cellule lymphatique et pouvant être cultivée dans un milieu sans sérum, le gène produisant ladite protéine exogène étant incorporé dans le myélome de souris avant la fusion cellulaire ou après la fusion cellulaire, on cultive ladite cellule fusionnée dans un milieu sans sérum pour l'expression du gène, et on recueille ladite protéine exogène à partir dudit milieu.

2. Procédé selon la revendication 1, dans lequel le myélome de souris est la lignée cellulaire P3X63Ag8.653.

3. Procédé selon la revendication 1, dans lequel la cellule lymphatique est une cellule hybride (hybridome) préparée à partir d'un myélome et d'une cellule lymphatique et pouvant être cultivée dans un milieu sans sérum.

4. Procédé selon la revendication 3, dans lequel la cellule hybride pouvant être cultivée dans un milieu sans sérum est la lignée cellulaire hybride Sp2/0.

5. Procédé selon l'une quelconque des revendications 1 à 4, dans lequel la protéine exogène est un anticorps recombiné.

6. Procédé selon la revendication 5, dans lequel la séquence d'aminoacides de l'anticorps recombiné comprend, au moins en tant qu'un de ses fragments, une séquence d'aminoacides dérivée d'un anticorps d'un animal autre qu'une souris.
